# EUROPEAN PATENT APPLICATION

(11) **EP 2 518 137 A1**
(43) Date of publication of application: **31.10.2012**
(21) Application number: 12165614.4
(22) Date of filing: 26.04.2012
(51) Int. Cl.: C12M 1/00

(54) **Isolator and method for moving culture**

(30) Priority: 28.04.2011 JP 2011102046
(71) Applicant: Panasonic Healthcare Co., Ltd., Ehime 791-0395 (JP)
(72) Inventor: Yokoi, Yasuhiko, Toon-shi, Ehime 791-0395 (JP); Kobayashi, Koichi, Toon-shi, Ehime 791-0395 (JP); Yamamoto, Hiroshi, Toon-shi, Ehime 791-0395 (JP); Busujima, Hiroki, Toon-shi, Ehime 791-0395 (JP)
(74) Representative: Charlton, Peter John

(57) **Abstract**

An isolator capable of having a culture apparatus demountably mounted thereto, the isolator includes: a working chamber; a storage chamber; a first opening configured to allow a culture chamber of the culture apparatus and the working chamber to communicate with each other, when the culture apparatus has been mounted; a second opening configured to allow the working chamber and the storage chamber to communicate with each other; a first door configured to close the second opening so as to hermetically seal the storage chamber; and a temperature adjusting device configured to, when culture taken out of the culture chamber has been stored in the storage chamber, and the storage chamber has been hermetically sealed, and thereafter the culture apparatus has been demounted, adjust temperature of the culture stored in the storage chamber.

## Description

### CROSS-REFERENCE TO RELATED APPLICATION

This application claims the benefit of priority to Japanese Patent Application No. 2011-102046, filed April 28, 2011, of which full contents are incorporated herein by reference.

### BACKGROUND OF THE INVENTION

### Field of the Invention

The present invention relates to an isolator and a method for moving culture.

### Description of the Related Art

In a series of cell culture processes, subculturing some of the cells or all of the cells in culture into a newly provided container is performed. Such work is called a passage, which is for example, performed in an isolator including a working chamber that is brought into communication with a culture chamber of a culture apparatus when the culture apparatus is mounted (see Japanese Laid-Open Patent Publication No. 2011-19762, for example).

For example, when the cells having been subcultured are recultured, in a case where there are a large number of containers storing the cells to be cultured, all of them may not be able to be stored in the culture apparatus. In such a case, it is required to demount the existing culture apparatus from the isolator, and mount on the isolator a newly provided culture apparatus having enough storage space. However in some cases, it takes time to demount the existing culture apparatus and provide a new culture apparatus before storing the cells therein, resulting in change in nature of the cells (culture) that were left in the isolator, which was originally unexpected.

### SUMMARY OF THE INVENTION

An isolator according to an aspect of the present invention, capable of having a culture apparatus demountably mounted thereto, includes: a working chamber; a storage chamber; a first opening configured to allow a culture chamber of the culture apparatus and the working chamber to communicate with each other, when the culture apparatus has been mounted; a second opening configured to allow the working chamber and the storage chamber to communicate with each other; a first door configured to close the second opening so as to hermetically seal the storage chamber; and a temperature adjusting device configured to, when culture taken out of the culture chamber has been stored in the storage chamber, and the storage chamber has been hermetically sealed, and thereafter the culture apparatus has been demounted, adjust temperature of the culture stored in the storage chamber.

Other features of the present invention will become apparent from descriptions of this specification and of the accompanying drawings.

### BRIEF DESCRIPTION OF THE DRAWINGS

For more thorough understanding of the present invention and advantages thereof, the following description should be read in conjunction with the accompanying drawings, in which:
Fig. 1 is a diagram illustrating a configuration of an isolator 10 according to a first embodiment of the present invention;
Fig. 2 is a flow chart showing an example of a process performed by a worker when culture to be subcultured is stored in a newly provided culture apparatus 16;
Fig. 3 is a diagram illustrating a state of an isolator 10 and a culture chamber A1 when passage is performed;
Fig. 4 is a diagram illustrating a state of an isolator 10 and a culture chamber A1 when passage has been finished;
Fig. 5 is a diagram illustrating a state of an isolator 10 when a chamber A1 is demounted;
Fig. 6 is a diagram illustrating a state of an isolator 10 and a culture chamber B1 when a working chamber 22 and a culture chamber B1 are subjected to a decontamination process;
Fig. 7 is a diagram illustrating a state of an isolator 10 when cases 110 and 111 are moved to a culture chamber B1;
Fig. 8 is a diagram illustrating main components of an isolator 11 according to a second embodiment of the present invention;
Fig. 9 is a diagram illustrating main components of an isolator 12 according to a third embodiment of the present invention.

### DETAILED DESCRIPTION OF THE INVENTION

At least the following details will become apparent from descriptions of this specification and of the accompanying drawings.

Sterilization means killing microorganisms establishing the highest degree of aseptic condition possible, however, in the present specification, it is assumed that sterilization includes so-called decontamination, decolonization, disinfection and the like. Further, an aseptic environment indicates an aseptic environment to the highest degree possible, a decontamination process indicates a process for realizing the aseptic environment, and a material to be used in the decontamination process is referred to as a decontamination material.

### == First embodiment ==

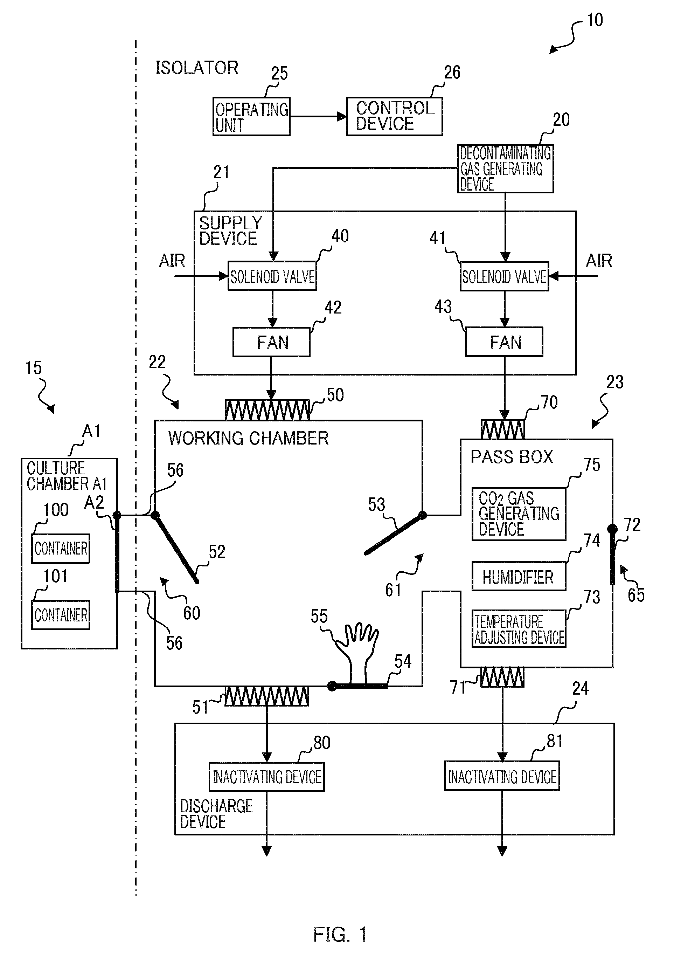

Fig. 1 is a diagram illustrating a configuration of an isolator 10 according to a first embodiment of the present invention. The isolator 10 is an apparatus for a worker to handle cells and the like in an aseptic environment. The culture apparatus 15 is an apparatus in which culture such as a cell is cultured, and includes: a culture chamber A1 configured to store containers 100 and 101 storing culture; and a door A2 configured to hermetically seal the culture chamber A1.

The culture apparatus 15 is mounted to the isolator 10, and the isolator 10 includes a decontaminating gas generating device 20, a supply device 21, a working chamber 22, a pass box 23, a discharge device 24, an operating unit 25, and a control device 26.

The decontaminating gas generating device 20 is configured to supply hydrogen peroxide gas, which is decontaminating gas, to the supply device 21.

The supply device 21 is a device configured to supply the hydrogen peroxide gas supplied thereto or air outside the isolator 10 to the working chamber 22 and the pass box 23, and includes solenoid valves 40, 41, and fans 42, 43.

The solenoid valves 40 and 41 are configured to respectively supply to the fans 42 and 43 the hydrogen peroxide gas or the outside air in a switching manner under the control of the control device 26. The fan 42 is configured to supply the hydrogen peroxide gas or the outside air through the solenoid valve 40 to the working chamber 22, and the fan 43 is configured to supply the hydrogen peroxide gas or the air through the solenoid valve 41 to the pass box 23. The decontaminating gas generating device 20 and the supply device 21 are equivalent to a decontamination material supply device.

The working chamber 22 is a space for working on a living organism-derived material such as a cell. The working chamber 22 is provided with air filters 50 and 51, doors 52 to 54, a working glove 55, and a coupling member 56. Further, the working chamber 22 is provided with an opening 60 on a wall face on the side where the culture apparatus 15 is mounted, and the working chamber 22 is provided with an opening 61 on a wall face on the side where the pass box 23 is provided.

The air filter 50 is a filter configured to remove dust and the like included in the hydrogen peroxide gas or the air supplied from the fan 42. The air filter 51 is a filter configured to remove dust and the like included in gas and the like discharged from the working chamber 22. A HEPA (High Efficiency Particulate Air) filter is used for the air filters 50 and 51, for example.

The door 52 is provided so as to be able to open or close the opening 60, and the door 53 is provided so as to be able to open or close the opening 61. Further, the door 54 is provided so as to be able to be opened or closed on a front side of the working chamber 22.

The working glove 55 is attached to an opening (not shown) provided on the door 54 so that a worker can work on a cell and the like in the working chamber 22 with the door 54 closed. The working chamber 22 is hermetically sealed when the doors 52-54 are closed.

The coupling member 56 is a member configured to connect the isolator 10 to the culture apparatus 15, for example, and is provided on an outer wall face of the working chamber 22 such that the opening 60 is surrounded. When the door 52 (second door) and the door A2 are opened in a case where the culture apparatus 15 is mounted to the coupling member 56, the working chamber 22 is brought into communication with the culture chamber A1 through the opening 60 (first opening). Further, when the door 53 (first door) is opened, the working chamber 22 is brought into communication with the pass box 23 through the opening 61 (second opening).

The pass box 23 is a space for sterilizing articles, such as culture equipment and culture medium, which are to be brought into the working chamber 22. For example, when the culture apparatus 15 is demounted, a container storing culture is temporarily stored in the pass box 23 (storage chamber).

The pass box 23 is provided with air filters 70 and 71, a door 72, a temperature adjusting device 73, a humidifier 74, and a carbon dioxide gas generating device 75.

The air filter 70 is configured to remove dust and the like included in the hydrogen peroxide gas or the air supplied from the fan 43, similar to the air filter 50, and the air filter 71 is configured to remove dust and the like included in gas and the like discharged from the pass box 23, similar to the air filter 51.

The door 72 (third door) is a door used when articles such as culture equipment and culture medium are brought into the working chamber 22. Although Fig. 1 illustrates a state where the door 72 is closed, articles such as culture equipment are brought in through an opening 65 (third opening) that allows communication between the pass box 23 and the exterior when the door 72 is opened. The pass box 23 is hermetically sealed when the doors 53 and 72 are closed.

The temperature adjusting device 73, the humidifier 74, and the carbon dioxide gas generating device (CO₂ gas generating device) 75 are configured to adjust temperature of a container storing culture or environment in the pass box 23, in order to prevent change in the nature of the culture when the culture is stored in the pass box 23.

The temperature adjusting device 73 is a plate-type thermostat, capable of having the container storing the culture placed thereon, using a Peltier device as the heat source, for example. The temperature adjusting device 73 is mounted on a floor of the pass box 23 and is configured to adjust temperature of the container storing the culture (temperature of the culture) to a predetermined temperature (37°C or 4°C) under the control of the control device 26, for example.

The humidifier 74 (humidity adjusting device) is configured to adjust the humidity in the hermetically sealed pass box 23 to a predetermined humidity (90%, for example) under the control of the control device 26.

The carbon dioxide gas generating device 75 (concentration adjusting device) is configured to generate CO₂ gas so as to adjust the concentration of CO₂ gas in the hermetically sealed pass box 23 to a predetermined concentration (5%, for example) under the control of the control device 26.

Thus, the pass box 23 according to an embodiment of the present invention realizes: a function of a storage chamber capable of storing culture with the culture being protected from change in the nature thereof; and functions of a sterilizing chamber, which are provided to a common isolator.

The discharge device 24 is a device configured to discharge gas, such as the hydrogen peroxide gas and the air, from the working chamber 22 and the pass box 23, and includes inactivating devices 80 and 81.

Each of the inactivating devices 80 and 81 includes a catalyst, and renders the gas to be harmless and outputs such harmless gas to the exterior of the isolator 10.

The operating unit 25 is an operating panel and the like for a worker to set an operation of the isolator 10. An operation result of the operating unit 25 is sent to the control device 26. Then, the control device 26 integrally controls the isolator 10 based on the operation result of the operating unit 25.

### == Process performed by worker when culture is moved to newly provided culture apparatus ==

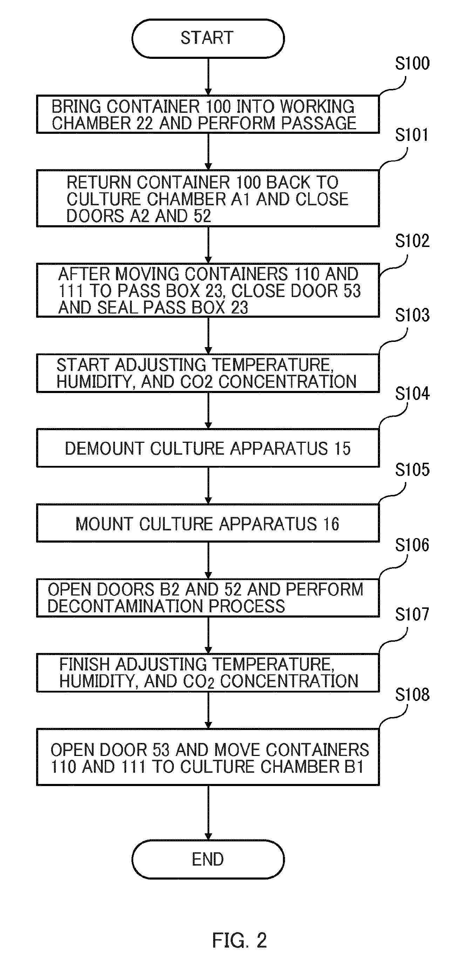

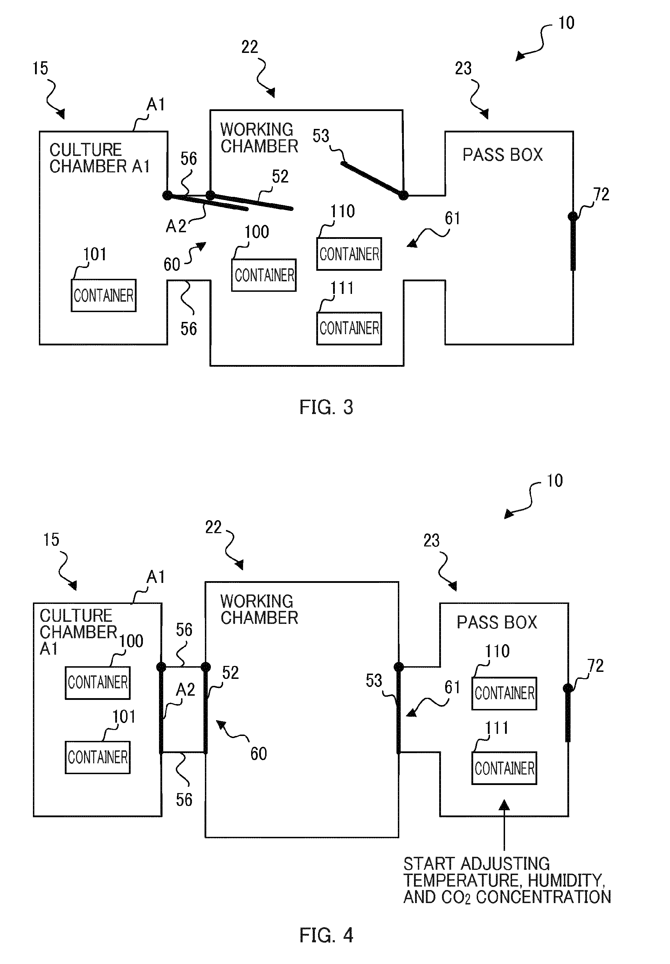

Here, with reference to Figs. 2 to 7, description will be given of a process performed by a worker when subcultured culture is stored into a newly provided culture apparatus 16 (second culture apparatus) in a case where the existing culture apparatus 15 (first culture apparatus) does not have enough storage space. When the culture apparatus 15 is mounted to the isolator 10, all of the culture chamber A1, the working chamber 22, and the pass box 23 are assumed to be in an aseptic environment, and the doors of the isolator 10 and the culture apparatus 15 are in a state as illustrated in Fig. 1, for example. Further, it is assumed that, as illustrated in Fig. 3, the containers 110 and 111 which are used when passage is performed are brought into the working chamber 22 beforehand. It should be noted that Figs. 3 to 6 illustrate only the main components in the isolator 10 for convenience' sake.

First, a worker opens the door A2 as illustrated in Fig. 3, takes the container 100 storing culture out from the culture chamber A1, to be brought into the working chamber 22, and performs passage (S100). Specifically, a worker moves a part of the culture (cells) to the containers 110 and 111.

Subsequently, when passage is finished, the worker returns the container 100 back to the culture chamber A1 and closes the door A2 and the door 52, as illustrated in Fig. 4 (S101). The worker moves the containers 110 and 111 to the pass box 23, and closes the door 53, thereby hermetically sealing the pass box 23 (S102: first and second processes).

After the pass box 23 is hermetically sealed, the worker operates the operating unit 25 so as to prevent change in nature of the culture, and starts adjusting the temperature of the containers 110 and 111 stored in the pass box 23, the humidity and the CO₂ concentration in the pass box 23 (S103: third process).

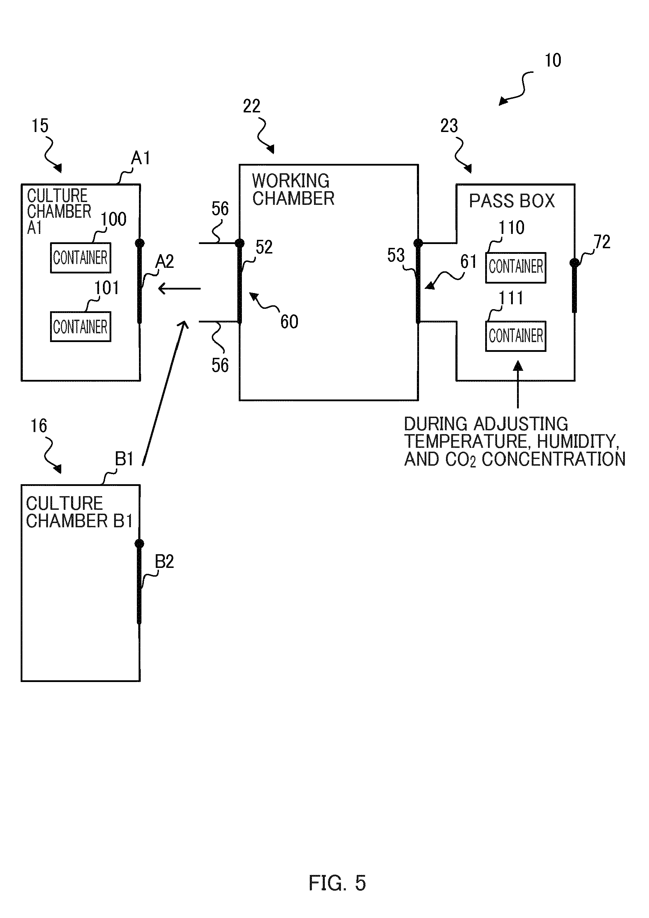

Then, the worker demounts the existing culture apparatus 15 as illustrated in Fig. 5 (S104: fourth process), and mounts the newly provided culture apparatus 16 to the isolator 10 (S105: fifth process). The culture apparatus 16 includes a culture chamber B1 and a door B2 configured to hermetically seal the culture chamber B1, similar to the culture apparatus 15. It is assumed here that no culture is stored in the culture chamber B1 and the interior of the culture chamber B1 has not been subjected to a decontamination process. Also, during this time period, the temperature and the like in the pass box 23 are kept adjusted. Thus, for example, even in a case where the processes S104 and S105 take time, it is possible to prevent change in nature of the culture stored in the cases 110 and 111.

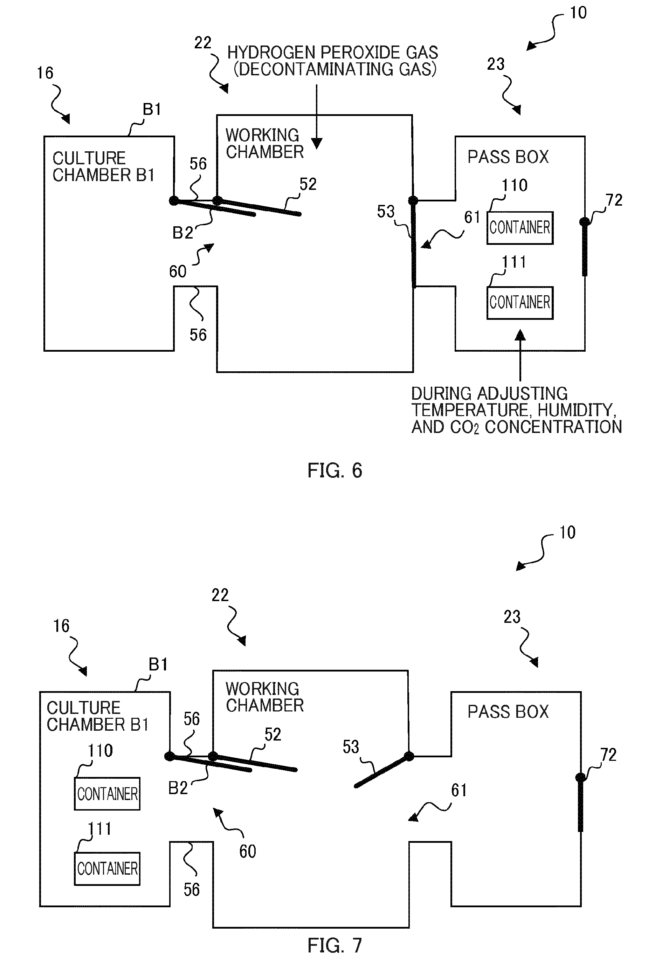

Then, the worker performs the decontamination process for sterilizing the coupling member 56 and the like that had been contaminated when the culture apparatus 15 was demounted, as well as sterilizing the interior of the culture chamber B1. Specifically, as illustrated in Fig. 6, the worker opens the door B2 and the door 52, and thereafter operates the operating unit 25, so as to allow the decontaminating gas generating device 20 and the like to perform the decontamination process (S106: sixth process). As a result, the hydrogen peroxide gas generated by the decontaminating gas generating device 20 is supplied to the working chamber 22 through the air filter 50. The hydrogen peroxide gas is also supplied to the culture chamber B1 through the working chamber 22, thereby sterilizing the working chamber 22 and the culture chamber B1. Since the pass box 23 is sealed during the decontamination process, the culture stored in the pass box 23 is not affected by the hydrogen peroxide gas.

Further, after the working chamber 22 and the culture chamber B1 are sterilized, a worker operates the operating unit 25, so as to finish adjusting the temperature, the humidity, and the CO₂ concentration (S107). Then, the worker, as illustrated in Fig. 7, opens the door 53 and moves the containers 110 and 111 stored in the pass box 23 to the culture chamber B1 (S108: seventh process). As a result, the culture stored in the newly provided containers 110 and 111 can be cultured in the culture chamber B1.

### == Second embodiment ==

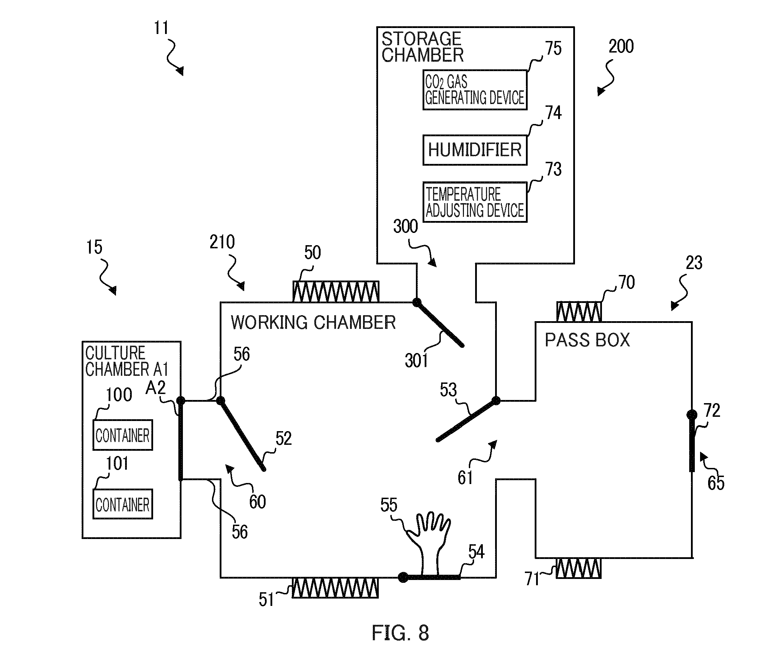

Fig. 8 is a diagram illustrating the main components of an isolator 11 according to a second embodiment of the present invention. Although the isolator 11 also includes the decontaminating gas generating device 20 and the like similar to the isolator 10 illustrated in Fig. 1, it is omitted here. The blocks illustrated in Fig. 8 that are equivalent to those illustrated in Fig. 1 are designated by the same reference numerals.

The isolator 11 includes a storage chamber 200 that is not included in the isolator 10 illustrated in Fig. 1. A working chamber 210 includes: an opening 300 (second opening) allowing communication between the storage chamber 200 and the working chamber 210; and a door 301 (first door) configured to hermetically seal the storage chamber 200 by closing the opening 300. The temperature adjusting device 73, the humidifier 74, and the CO₂ gas generating device 75 are provided in the storage chamber 200. Thus, for example, when the culture apparatus 15 is demounted, a worker can retain a container storing culture in the storage chamber 200. Then, the environment (temperature, humidity and the like) of the storage chamber 200 is adjusted, thereby preventing change in nature of the culture stored in the storage chamber 200.

### == Third embodiment ==

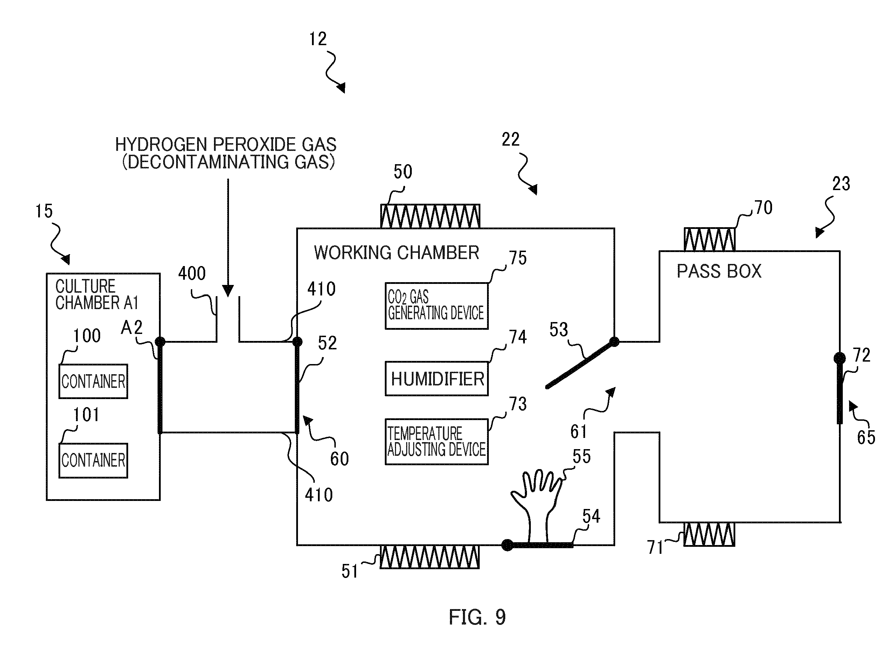

Fig. 9 is a diagram illustrating the main components of an isolator 12 according to a third embodiment of the present invention. Although not shown, the isolator 12 also includes the decontaminating gas generating device 20 and the like similar to the isolator 10 illustrated in Fig. 1. The blocks illustrated in Fig. 9 that are equivalent to those illustrated in Fig. 1 are designated by the same reference numerals.

When comparing the isolator 12 with the isolator 10, the isolator 12 is provided with a coupling member 410, including a port 400 through which hydrogen peroxide gas is supplied, in place of the coupling member 56. Such a coupling member 410 is disclosed in Japanese Laid-Open Patent Publication No. 2011-19762, for example.

The temperature adjusting device 73, the humidifier 74, and the CO₂ gas generating device 75 are provided in the working chamber 22. Thus, for example, when the culture apparatus 15 is demounted, a worker can retain a container storing culture in the working chamber 22 with the doors 52 and 53 closed. Then, the environment (temperature, humidity and the like) of the working chamber 22 is adjusted, thereby preventing change in nature of the culture stored in the working chamber 22.

Hereinabove, descriptions have been given of the isolators 10 to 12 according to the embodiments of the present invention. In the isolator 10, culture is stored in the pass box 23 that is provided with the temperature adjusting device 73, when the culture apparatus 15 is demounted. Thus, even when the culture is temporarily kept in the isolator 10, it is possible to prevent change in nature of the culture.

Further, the isolator 10 is provided with the door 52 that is configured to close the opening 60 when the culture apparatus 15 is demounted. Thus, it is possible to suppress contamination of the working chamber 22 and leak of substance from the working chamber 22 to the exterior, when the culture apparatus 15 is demounted.

Further, in the isolator 10, the pass box 23 to which the articles are brought in from the exterior is also used as a storage chamber to temporarily store the culture. Thus, it is possible to save space.

Further, the pass box 23 in the isolator 10 includes the carbon dioxide gas generating device 75. Thus, it is possible to prevent change in nature of the culture as compared with a case where only the temperature of the culture is adjusted.

Further, the pass box 23 in the isolator 10 includes the humidifier 74. Thus, it is possible to prevent change in nature of the culture as compared with a case where only the temperature of the culture is adjusted.

Further, in a case where only the coupling member 410 can be decontaminated as in the isolator 12, for example, the culture may be temporarily kept in the working chamber 22 provided with the temperature adjusting device 73. Even in such a case, it is possible to prevent change in nature of the culture.

Further, in the isolator 10, when the newly provided culture apparatus 16 is mounted, the culture chamber B1 and the working chamber 22 can be sterilized together. Thus, when the newly provided culture apparatus 16 is mounted, it is possible to save the effort of decontaminating the culture chamber B1 beforehand.

It was assumed, for example, that the temperature adjusting device 73 is a plate-type thermostat, using a Peltier device as a heat source, which is capable of directly adjusting the temperature of the container storing the culture, however, it is not limited thereto. The temperature adjusting device 73 may be a device configured to adjust the temperature in the pass box 23 in the isolator 10, for example. When the temperature in the pass box 23 is adjusted to 37°C, for example, the temperature of the culture stored in the pass box 23 results in substantially the same temperature. Thus, even in such a case, it is possible to prevent the change in nature of the culture. Further, the device may be a heater and the like mounted on a wall on the outside of the pass box 23, for example, as long as the temperature adjusting device 73 is capable of adjusting the temperature in the pass box 23. That is to say, the temperature adjusting device 73 need not be provided on the inside of the pass box 23.

In a case where a part of the working chamber 22 is space partitioned by a hermetically sealing door, if the temperature and the like of the partitioned space can be adjusted, culture can be stored while suppressing change in nature of the culture. The space formed by partitioning the chamber, where temperature and the like can be adjusted may be provided under the floor of the working chamber 22.

The above embodiments of the present invention are simply for facilitating the understanding of the present invention and are not in any way to be construed as limiting the present invention. The present invention may variously be changed or altered without departing from its spirit and encompass equivalents thereof.

## Claims

1. An isolator capable of having a culture apparatus demountably mounted thereto, the isolator comprising:
a working chamber;
a storage chamber;
a first opening configured to allow a culture chamber of the culture apparatus and the working chamber to communicate with each other, when the culture apparatus has been mounted;
a second opening configured to allow the working chamber and the storage chamber to communicate with each other;
a first door configured to close the second opening so as to hermetically seal the storage chamber; and
a temperature adjusting device configured to, when culture taken out of the culture chamber has been stored in the storage chamber, and the storage chamber has been hermetically sealed, and thereafter the culture apparatus has been demounted, adjust temperature of the culture stored in the storage chamber.

2. The isolator according to claim 1, further comprising
a second door configured to close the first opening when the culture apparatus is demounted,

3. The isolator according to claim 2, further comprising:
a third opening configured to allow the storage chamber and exterior to communicate with each other; and
a third door configured to open or close the third opening,
wherein the third door is opened, when the second opening has been closed by the first door and an article is brought into the storage chamber.

4. The isolator according to claim 1, further comprising
a concentration adjusting device configured to, when the culture taken out of the culture chamber has been stored in the storage chamber, and the storage chamber has been hermetically sealed, and thereafter the culture apparatus has been demounted, adjust carbon dioxide concentration in the storage chamber.

5. The isolator according to claim 2, further comprising
a concentration adjusting device configured to, when the culture taken out of the culture chamber has been stored in the storage chamber, and the storage chamber has been hermetically sealed, and thereafter the culture apparatus has been demounted, adjust carbon dioxide concentration in the storage chamber.

6. The isolator according to claim 3, further comprising
a concentration adjusting device configured to, when the culture taken out of the culture chamber has been stored in the storage chamber, and the storage chamber has been hermetically sealed, and thereafter the culture apparatus has been demounted, adjust carbon dioxide concentration in the storage chamber.

7. The isolator according to claim 1, further comprising
a humidity adjusting device configured to, when the culture taken out of the culture chamber has been stored in the storage chamber, and the storage chamber has been hermetically sealed, and thereafter the culture apparatus has been demounted, adjust humidity in the storage chamber.

8. The isolator according to claim 2, further comprising
a humidity adjusting device configured to, when the culture taken out of the culture chamber has been stored in the storage chamber, and the storage chamber has been hermetically sealed, and thereafter the culture apparatus has been demounted, adjust humidity in the storage chamber.

9. The isolator according to claim 3, further comprising
a humidity adjusting device configured to, when the culture taken out of the culture chamber has been stored in the storage chamber, and the storage chamber has been hermetically sealed, and thereafter the culture apparatus has been demounted, adjust humidity in the storage chamber.

10. The isolator according to claim 4, further comprising
a humidity adjusting device configured to, when the culture taken out of the culture chamber has been stored in the storage chamber, and the storage chamber has been hermetically sealed, and thereafter the culture apparatus has been demounted, adjust humidity in the storage chamber.

11. An isolator capable of having a culture apparatus demountably mounted thereto, the isolator comprising:
a working chamber;
an opening configured to allow a culture chamber of the culture apparatus and the working chamber to communicate with each other, when the culture apparatus has been mounted;
a door configured to close the opening so as to hermetically seal the working chamber; and
a temperature adjusting device configured to , when culture taken out of the culture chamber has been stored in the working chamber, and the working chamber has been hermetically sealed, and thereafter the culture apparatus has been demounted, adjust temperature of the culture stored in the working chamber.

12. A method for moving culture stored in a first culture chamber of a first culture apparatus to a second culture chamber of a second culture apparatus in an isolator to which the first culture apparatus or the second culture apparatus can be mounted, the isolator including: a working chamber; a storage chamber; a first opening configured to allow the first or the second culture chamber and the working chamber to communicate with each other; a second opening configured to allow the working chamber and the storage chamber to communicate with each other; a door configured to close the second opening so as to hermetically seal the storage chamber; a decontamination material supply device configured to supply a decontamination material into the working chamber; and a temperature adjusting device included in the storage chamber, the method comprising:
a first process of moving the culture in the first culture chamber to the storage chamber, when the first culture apparatus has been mounted;
a second process of closing the door and hermetically sealing the storage chamber;
a third process of causing the temperature adjusting device to adjust temperature of the culture;
a fourth process of demounting the first culture apparatus;
a fifth process of mounting the second culture apparatus;
a sixth process of causing the decontamination material supply device to decontaminate the working chamber and the second culture chamber; and
a seventh process of opening the door and moving the culture stored in the storage chamber to the second culture chamber.
